# EUROPÄISCHE PATENTANMELDUNG

(11) **EP 0 722 703 A2**
(43) Veröffentlichungstag der Anmeldung: **24.07.1996**
(21) Anmeldenummer: 95120754.7
(22) Anmeldetag: 30.12.1995
(51) Int. Cl.: A61F 2/32

(54) **Künstliches Gelenk, insbesondere Hüftgelenk-Endoprothese**

(30) Priorität: 21.01.1995 DE 19501771
(71) Anmelder: Aldinger, Günther, Dr. med., D-72076 Tübingen (DE)
(72) Erfinder: Aldinger, Günther, Dr. med., D-72076 Tübingen (DE)
(74) Vertreter: Möbus, Rudolf, Dipl.-Ing.

(57) **Zusammenfassung**

Bei dem künstlichen Gelenk mit einer Kontaktflächenpaarung Metall/Metall, Keramik/Keramik oder Keramik/Metall ist der Randbereich (15.2) der Gelenkpfanne (15) aus einem weicheren Material als der Gelenkkopf gefertigt, so daß der Gelenkpfannenrand auch in extremen Gelenkstellungen keinen Abrieb an dem Gelenkkopf erzeugen kann.

## Beschreibung

Die Erfindung betrifft ein künstliches Gelenk, insbesondere eine Hüftgelenk-Endoprothese, mit einer Gelenkpfanne und einem Gelenkkopf als ineinanderpassende Gelenkpartner, deren Gelenk-Kontaktflächen aus Metall oder Keramik bestehen.

Hüftgelenk-Endoprothesen, bei denen es neben der Schmerzlinderung für den Patienten und der Verbesserung seiner Beweglichkeit auch darum geht, eine möglichst lange Lebensdauer der Implantate zu erreichen, sind auch mit den vorstehend genannten Materialkombinationen der Gelenkpartner bekannt. Klinisch gängige Hüftsysteme und auch andere künstliche Gelenke bestehen jedoch überwiegend aus einer Polyäthylen/Metall-Paarung. Auch hat sich die Paarung eines Keramik-Gelenkkopfes in einer Pfanne aus Kunststoffmaterial bewährt. Zwar sind hochmolekulare Kunststoffmaterialien gut körperverträglich und weisen gute Gleitverhältnisse auf, doch ist nicht zu überschen, daß bei künstlichen Gelenken, wie Hüftgelenk-Endoprothesen, sehr hohe Belastungswerte bei hoher Lastwechselzahl und wechselnden Belastungsrhythmen auftreten und sich die Beanspruchungsdauer über Jahrzehnte erstrecken soll. Hierbei kann nicht immer von einer gleichmäßigen Gelenkschmierung ausgegangen werden, so daß die Gelenkreibung ebenfalls eine merkliche Rolle spielt, die auch bei hochmolekularen Kunststoffmaterialien zu einem nicht vernachlässigbaren Abrieb führt. Die entstehenden Abriebteilchen werden ab einer bestimmten Größe des Abriebvolumens von dem das künstliche Gelenk umgebenden Weichteilgewebe nicht mehr reizlos vertragen, und in der Fachliteratur wird insbesondere in der jüngeren Zeit immer mehr über die Bildung von Fremdkörpergranulomen und knöchernen Osteolysen aufgrund von Abriebpartikeln geschrieben.

Es ist bekannt, daß bei Kontaktflächenpaarungen Metall/Metall und insbesondere Keramik/Keramik ein wesentlich geringerer Abrieb an den Gelenkpartnern auftritt. Trotz dieser geringen Verschleißwerte konnten sich die Keramik/Keramik- und auch Metall/Metall-Paarungen klinisch noch nicht ausreichend durchsetzen. Verbesserte Herstellungsmethoden, die zu hoher Formgenauigkeit und Kontaktflächengüte mit sehr geringer Rauhtiefe führen, sowie Materialverbesserungen haben die Abriebfestigkeit der Gelenkteile inzwischen noch weiter verbessert. Hinzu kommt, daß Metalle und insbesondere Keramikmaterial mit Körperflüssigkeit gut benetzbar sind. Auch läßt sich die niedrigere Schlag/Biege-Festigkeit von Keramikmaterial durch eine stoßdämpfende Lagerung der Gelenkpfanne in einem Bett aus Kunststoffmaterial berücksichtigen. Die trotzdem bestehende Zurückhaltung beim klinischen Einsatz der genannten Materialpaarungen ist dennoch nicht unbegründet, da man Labortestergebnisse nicht schlechthin auch auf klinische Ergebnisse übertragen darf, und es haben sich in der Tat bei explantierten Keramik-Gelenkköpfen von Kunstgelenken mit beiderseitigen Keramik-Kontaktflächen Abriebmuster gezeigt, die folgenden Verschleißmechanismus vermuten lassen:
Beim Überschreiten des freien Bewegungsradius des Kunstgelenkes, also bei einem Heraushebeln des Gelenkkopfes durch Anschlagen des Gelenkhalses am Rande der Gelenkpfanne, wie auch bei einem teilweisen Herausgleiten des Gelenkkopfes aus der Gelenkpfanne bei vermindertem Muskeltonus, wie er beispielsweise im Schlaf auftritt, kommt es zu einer erhöhten Reibung des Gelenkkopfes an dem harten, teils rauhen Schalenrand, sowie zu einem Abbruch des Schmierflüssigkeitsfilmes.

Der Erfindung liegt nun die Aufgabe zugrunde, ein künstliches Gelenk der eingangs genannten Art so auszubilden, daß auch bei einer Subluxation oder bei einem teilweisen Herausgleiten des Gelenkkopfes aus der Gelenkpfanne bei vermindertem Muskeltonus ein Abrieb an den Gelenkpartnern nicht auftreten kann.

Die gestellte Aufgabe wird erfindungsgemäß dadurch gelöst, daß der noch eine Kontaktfläche für den Gelenkkopf bildende Pfannenrand aus einem anderen abriebfesten und körperverträglichen Material besteht, das weicher als das die Kontaktoberfläche des Gelenkkopfes bildende Material ist.

Bei einem erfindungsgemäß ausgebildeten künstlichen Gelenk kann auch in den vorstehend genannten Gelenkstellungen kein Abrieb an dem Gelenkkopf erfolgen, an welchem auftretender Abrieb sich besonders nachteilig auswirken würde. Dabei kann der weichere Gelenkpfannenrand zweckmäßig aus einem hochmolekularen Kunststoffmaterial bestehen, wie es von klinisch gängigen künstlichen Gelenken her bekannt ist. Da der Gelenkpfannenrand bei einem künstlichen Gelenk insgesamt weniger reibungsbelastet ist, muß hier beim Einsatz von Kunststoffmaterial nicht mit einer merklichen Erhöhung von Abriebsvolumen gerechnet werden. Es sind aber auch Metallegierungen als vergleichsweise weicheres Material für den Gelenkpfannenrand bei Gelenken mit einer Kontaktflächenpaarung Keramik/Keramik zur Lösung der gestellten Aufgabe möglich.

Vorteilhafterweise kann aus dem den Gelenkpfannenrand bildenden weicheren Material ein in einem Haltekörper der Gelenkpfanne verankerbarer Zwischenkörper geformt sein, in welchen die die Gelenk-Kontaktfläche bildende Gelenkpfanne aus Keramik oder Metall eingesetzt und durch einen einwärts vorspringenden Kontaktflächenrand des Zwischenkörpers in seiner Lage formschlüssig gesichert ist. Der weichere Kontaktflächenrand der Gelenkpfanne kann zweckmäßig durch einen durchgehenden Umfangsrandsteg gebildet sein. Dieser Umfangsrandsteg kann aber auch mindestens kontaktflächenseitig Unterbrechungen oder Vertiefungen aufweisen, welche ein unerwünschtes Abreißen des Schmiermittelfilmes im Randbereich erschweren und in denen sich auch eventuelle Abriebpartikel sammeln können. In jedem Falle kann es vorteilhaft sein, den weicheren Kontaktflächenrand der Gelenkpfanne mit einer gebrochenen oder abgerundeten Außenkante zu versehen, die eine Anschlagfläche für den Gelenkkopf oder einen anschließenden Gelenkhals bildet. An der Übergangsstelle zu der übrigen Kontaktfläche der Gelenkpfanne kann der weichere Kontaktflächenrand ebenfalls besonders gestaltet sein, beispielsweise mit einer schrägen, hinterschnittenen Innenkante zur Verbesserung der formschlüssigen Halterung der eigentlichen Gelenkpfanne versehen sein. Die Innenkante kann aber auch eine nicht hinterschneidende Abschrägung aufweisen, welche ein nachträgliches Einsetzen der eigentlichen Gelenkpfanne in das den Gelenkpfannenrand bildende weichere Material, beispielsweise durch Einklipsen der Pfanne in einen aus diesem weicheren Material gebildeten Haltekörper, begünstigt.

Nachfolgend wird ein Ausführungsbeispiel des Erfindungsgegenstandes anhand der beiliegenden Zeichnung näher erläutert.

Im einzelnen zeigen:
- Fig. 1: eine schematisierte Gesamtdarstellung einer Hüftgelenk-Endoprothese mit bereits in dem Oberschenkelknochen verankerten Gelenkkopf;
- Fig. 2: einen Querschnitt durch die Gelenkpfanne der Endoprothese;
- Fig. 3 bis 5: vergrößerte Teilschnittdarstellungen des in Fig. 2 mit X bezeichneten, unterschiedlich ausgebildeten Randbereiches der Gelenkpfanne.

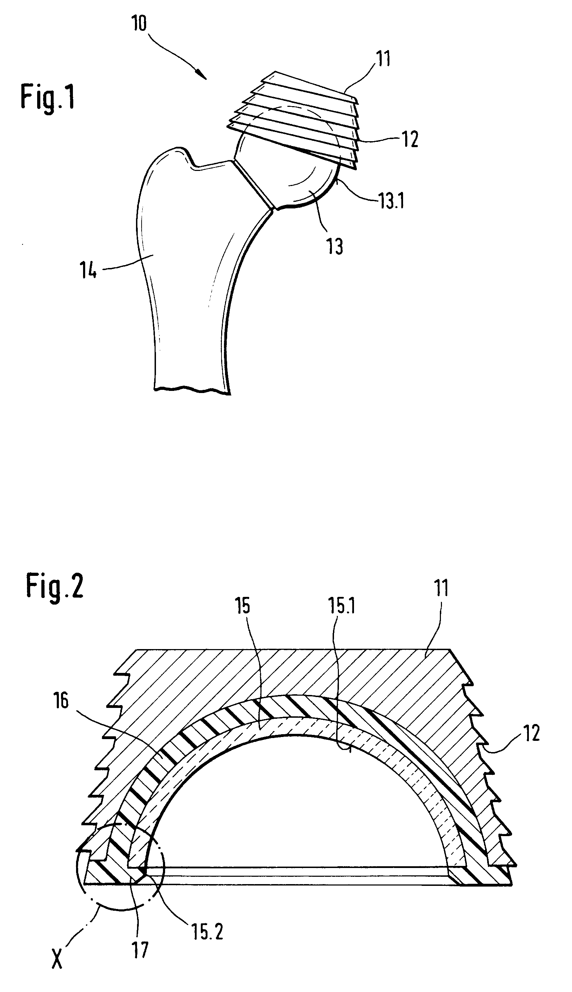

Fig. 1 zeigt von einer Hüftgelenk-Endoprothese 10 einen als Schraubkörper ausgebildeten und mit einem Außengewinde 12 verschenen Haltekörper 11 für die Gelenkpfanne und den mit der Gelenkpfanne als Gelenkpartner zusammenwirkenden kugelförmigen Gelenkkopf 13, der mit einem nicht dargestellten Schaft bereits in einem Oberschenkelknochen 14 verankert ist. Beide Gelenkpartner, die aus Fig. 2 ersichtliche Gelenkschale 15 und der Gelenkkopf 13, sind aus sogenanntem Biokeramikmaterial, z. B. Zirkonoxyd, gefertigt und an ihren Kontaktflächen 15.1 und 13.1 formgenau und mit sehr geringer Rauhtiefe geschliffen und poliert. Die Paßgenauigkeit des Gelenkkopfes 13 in die Gelenkpfanne 15 ist so getroffen, daß sich ein Schmierfilm von Körperflüssigkeit zwischen den Gelenkpartnern ausbilden und halten kann.

Wie aus dem Schnittbild der Fig. 2 ersichtlich ist, ist die Gelenkschale 15 in einen Zwischenkörper 16 eingesetzt, der in dem Haltekörper 11 befestigt ist und beispielsweise aus einem hochmolekularen Kunststoffmaterial besteht. Dieser Zwischenkörper 16 bewirkt einerseits eine begrenzt elastische Lagerung der Keramik-Gelenkpfanne 15 in dem Haltekörper 11 und bildet anderseits mit einem einwärts vorspringenden Ringsteg 17 den noch einen Teil der Kontaktfläche der Gelenkpfanne 15 bildenden Gelenkpfannenrand 15.2. Die Keramik-Kontaktfläche 15.1 der Gelenkpfanne 15 geht also stufenlos in den aus dem weicheren Material gefertigten Kontaktflächenrand 15.2 über.

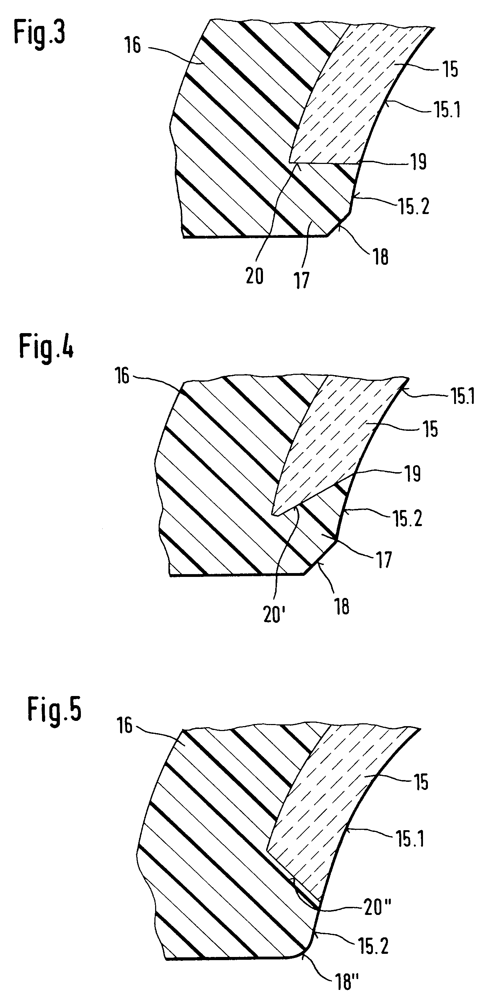

Der einwärts vorspringende Ringsteg 17 des Zwischenkörpers 16 kann ein unterschiedliches Querschnittsprofil haben, wie die Fig. 3 bis 5 zeigen. In allen Ausführungsformen der Fig. 3 bis 5 sind die Abmessungen so gewählt, daß die sphärische Gelenkpfanne 15 einschließlich des Ringsteges 17 etwas kleiner als eine Halbkugel ist, also sich über einen Kugelwinkelbereich von etwas kleiner als 180° erstreckt. In den Ausführungsbeispielen nach Fig. 3 und 4 weist der Ringsteg 17 unter Bildung einer Anschlagfläche 18 für einen Gelenkkopfhals eine gebrochene Außenkante auf. Bei dem Ausführungsbeispiel nach Fig. 5 ist die Außenkante mit einer Abrundung 18'' versehen. In allen Fällen bildet der Ringsteg 17 einen sphärischen Kontaktflächenrand 15.2 und geht mit der Innenkante 19 des Ringsteges 17 stufenlos in die Kontaktfläche 15.1 der Keramik-Gelenkschale 15 über. Der Unterschied bei den Ausführungsbeispielen besteht darin, daß bei dem Ausführungsbeispiel nach Fig. 3 die Innenseite 20 des Ringsteges 17 und die gegen diese Innenseite 20 anliegende Stirnfläche der Gelenkpfanne 15 in einer Kugelsektorebene verlaufen, daß bei dem Ausführungsbeispiel nach Fig. 4 die Innenseite 20' einen Hinterschnitt begrenzt, in welchen der entsprechend geformte stirnseitige Abschluß der Gelenkpfanne 15 passend eintaucht und daß bei dem Ausführungsbeispiel nach Fig. 5 die Innenseite 20'' schräg verläuft, ohne einen Hinterschnitt zu bilden. In allen drei Ausführungsfällen ist die Gelenkpfanne 15 in dem weicheren Zwischenkörper 16 formschlüssig verankert. Die Ausführungsform nach Fig. 5 erleichtert ein Einklipsen der vorgefertigten Gelenkpfanne 15 in einen vorgefertigten Zwischenkörper 16. Hierzu können die Innenseite 20'' und dementsprechend auch die Stirnfläche der Gelenkpfanne 15 auch in einer Kugeldurchmesserebene verlaufen.

Der Ringsteg 17 kann im Bereich seiner Außenkante auch profiliert sein, etwa dergestalt, daß durch einzelne gerundete Erhebungen oder durch Vertiefungen im Kantenbereich nur kleine Anlageflächen für den Gelenkkopf bei Subluxation vorliegen.

## Patentansprüche

1. Künstliches Gelenk, insbesondere Hüftgelenk-Endoprothese, mit einer Gelenkpfanne (15) und einem Gelenkkopf (13) als ineinanderpassende Gelenkpartner, deren Gelenk-Kontaktflächen (15.1, 13.1) aus Metall oder Keramik bestehen, dadurch gekennzeichnet, daß der noch eine Kontaktfläche (15.2) für den Gelenkkopf (13) bildende Pfannenrand aus einem anderen abriebfesten und körperverträglichen Material besteht, das weicher als das die Kontaktoberfläche (13.1) des Gelenkkopfes (13) bildende Material ist.

2. Künstliches Gelenk nach Anspruch 1, dadurch gekennzeichnet, daß der weichere Gelenkpfannenrand (Ringsteg 17) aus einem hochmolekularen Kunststoffmaterial besteht.

3. Künstliches Gelenk nach Anspruch 1 oder 2, dadurch gekennzeichnet, daß aus dem den Gelenkpfannenrand (17) bildenden weicheren Material ein in einem Haltekörper (11) der Gelenkpfanne verankerbarer Zwischenkörper (16) geformt ist, in welchen die die Gelenk-Kontaktfläche (15.1) bildende Gelenkpfanne (15) aus Keramik oder Metall eingesetzt und durch einen einwärts vorspringenden Kontaktflächenrand (Ringsteg 17) des Zwischenkörpers (16) in seiner Lage formschlüssig gesichert ist.

4. Künstliches Gelenk nach einem der Ansprüche 1 bis 3, dadurch gekennzeichnet, daß der weichere Kontaktflächenrand (15.2) der Gelenkpfanne (15) durch einen durchgehenden Umfangsrandsteg (17) gebildet ist.

5. Künstliches Gelenk nach einem der Ansprüche 1 bis 3, dadurch gekennzeichnet, daß der weichere Kontaktflächenrand (15.2) der Gelenkpfanne (15) durch einen mindestens kontaktflächenseitig Unterbrechungen oder Vertiefungen aufweisenden Umfangsrandsteg gebildet ist.

6. Künstliches Gelenk nach einem der Ansprüche 1 bis 5, dadurch gekennzeichnet, daß der weichere Kontaktflächenrand (15.2) der Gelenkpfanne (15) mit einer gebrochenen, eine Anschlagfläche (18) bildenden Außenkante versehen ist.

7. Künstliches Gelenk nach einem der Ansprüche 1 bis 6, dadurch gekennzeichnet, daß der weichere Kontaktflächenrand (15.2) an der Übergangsstelle (Innenkante 19) zu der übrigen Kontaktfläche (15.1) der Gelenkpfanne (15) mit einer hinterschnittenen Innenseite (20') versehen ist (Fig. 4).

8. Künstliches Gelenk nach einem der Ansprüche 1 bis 7, dadurch gekennzeichnet, daß die Kontaktfläche (15.1) der Gelenkpfanne (15) sich über einen Kugelwinkelbereich erstreckt, der kleiner als 180°, also kleiner als eine Halbkugel ist.
